# EUROPEAN PATENT SPECIFICATION

(11) **EP 1 829 127 B1**
(45) Date of publication and mention of the grant of the patent: **15.09.2010**
(21) Application number: 05851342.5
(22) Date of filing: 01.11.2005
(51) Int. Cl.: H01L 41/08, A61B 8/00, H01L 41/22, A61B 18/14, B06B 1/06

(54) **PIEZOCOMPOSITE TRANSDUCERS**
PIEZOVERBUNDWANDLER
TRANSDUCTEURS PIEZOCOMPOSITES

(30) Priority: 08.11.2004 US 984664
(43) Date of publication of application: 05.09.2007
(73) Proprietor: Boston Scientific Limited, Hastings Christ Church (BB)
(72) Inventor: YUAN, Jian, R., Hayward, California 94544 (US); CAO, Pei, J., Fremont, California 94539 (US); ROMLEY, Richard, Camation, Washington 98014-8236 (US)
(74) Representative: Golkowsky, Stefan
(86) International application number: PCT/US2005/039848
(87) International publication number: WO 2006/052685

(56) References cited:
- EP-A- 0 355 694
- US-A- 4 422 003
- US-A- 4 572 981
- US-A- 4 683 396
- US-A- 4 933 230
- US-A- 5 957 851
- US-A- 6 036 647
- US-A- 6 159 149
- US-B1- 6 277 299

## Description

### FIELD OF THE INTENTION

The systems described herein relate to the fabrication, implementation and use of piezocomposite transducers in intravascular, intracardiac and similar ultrasound imaging systems.

### BACKGROUND INFORMATION

Many diagnostic and therapeutic advantages exist in medical imaging systems that image the interior of a living being using an imaging device insertable into the living being. Examples of such systems include intravascular ultrasound (NUS) imaging systems, intracardiac echocardiography (ICE) imaging systems and the like. These systems can be used in many applications, such as locating and treating plaque buildup within the carotoid or coronary arteries of the patient, imaging the chambers of the heart, or blood vessels and the like. Transducers used within these imaging devices are typically formed from strictly piezoceramic materials. However, these materials have significant disadvantages.

One disadvantage is that piezoceramic materials typically have an acoustic impedance much higher than the surrounding environment. For instance, in some case the acoustic impedance of a piezoceramic transducer is higher than 30 MRayl, while the surrounding environment, such as blood or soft tissue, is on the order of 1.5 MRayl. This results in a significant acoustic impedance mismatch which typically requires the use of additional matching layers around the transducer to lessen the severity of the mismatch. However, these additional matching layers can prevent the transducer from achieving optimum performance.

Another disadvantage is that piezoceramic materials have limited ultrasound bandwidth and sensitivity. The bandwidth and sensitivity of a transducer is directly affected by the electric-mechanical coupling coefficient of the material used to fabricate the transducer. In most medical ultrasound applications, the transducers are fabricated as a plate and use a thickness mode of operation. In these applications, the electric-mechanical coupling coefficient, kₜ, is approximately 0.5. This low coefficient severely limits the bandwidth and sensitivity of the transducer, resulting in degraded imaging performance.

Furthermore, piezoceramic materials tend to be quite fragile. This can prevent the piezoceramic material from being shaped or configured in the most optimal manner. For instance, the fragility of piezoceramic materials can prevent the transducer from being shaped to focus the transducer on a desired range of depths.

Document US-A-4 572 981 discloses a device as described in the preamble of claim 1.

Accordingly, there is a need for a transducer capable of overcoming these and other disadvantages and allowing improved performance over transducers formed from strictly piezoceramic materials.

### SUMMARY

The embodiments described herein provide for an imaging system having an imaging device preferably insertable within a living being and configured to image the living being with a piezocomposite transducer. The piezocomposite transducer can be configured in any manner in accordance with the needs of the application. In example embodiments, the piezocomposite transducer is configured as a single element transducer, and multiple array configurations and types, including linear arrays, phased arrays, one dimensional array, two dimensional arrays, arrays having one or more rows with one or more transducers in each row, annular arrays and other arrays. Also, the piezocomposite transducer can be shaped in any manner in accordance with the needs of the application. The piezocomposite transducer preferably includes a piezoceramic material and a polymeric material. The piezoceramic material and polymeric material can be arranged in any configuration in accordance with the needs of the application.

Other systems, features and advantages of the invention will be or will become apparent to one with skill in the art upon examination of the following figures and detailed description. It is intended that all such additional systems, features and advantages be included within this description, be within the scope of the invention, and be protected by the accompanying claims. It is also intended that the invention is not limited to require the details of the example embodiments.

### BRIEF DESCRIPTION OF THE FIGURES

The details of the invention, including fabrication, structure and operation, may be gleaned in part by study of the accompanying figures, in which like reference numerals refer to like segments.
FIG. 1 is a schematic view depicting an example embodiment of a medical device having a piezocomposite transducer.
FIGs. 2-4 are perspective views of example embodiments of piezocomposite materials having various configurations.
FIGs. 5A-C are perspective views of example embodiments ofpiezocomposite transducers configured as single element transducers.
FIGs. 6A-7B are perspective views of example embodiments of piezocomposite transducers configured as transducer arrays.
FIG. 8 is a graph of the performance characteristics of an example embodiment of piezocomposite material.
FIGs. 9A-B are graphs of the impulse response for a piezoceramic transducer and piezocomposite transducer, respectively.
FIG. 10 is a flow diagram depicting an example method of manufacturing a piezocomposite transducer.
FIGs. 11-12 are schematic views of example embodiments of imaging systems having piezocomposite transducers.
FIG. 13 is a flow diagram depicting an example method of imaging with a piezocomposite transducer.

### DETAILED DESCRIPTION

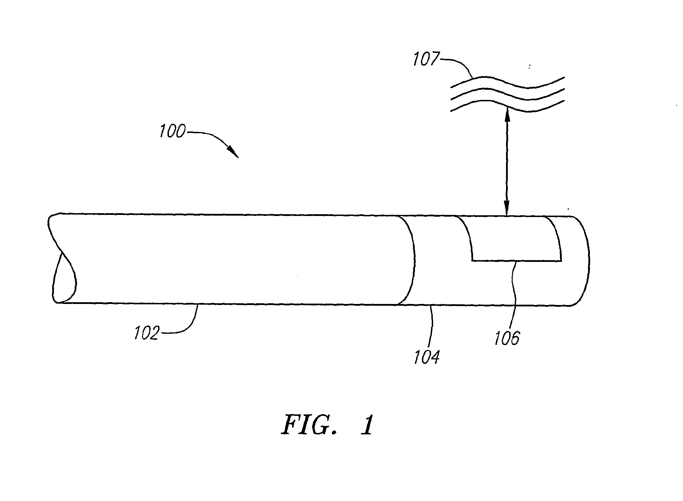

The systems described herein provide for ultrasound imaging devices configured for imaging with piezocomposite materials. FIG. 1 depicts one example embodiment of an ultrasound imaging system 100 having an elongate medical device 102 configured for insertion into the body of a living being. The elongate medical device 102 includes an imaging device 104 having a piezocomposite transducer 106 for imaging tissue 107 within the interior of the living being.

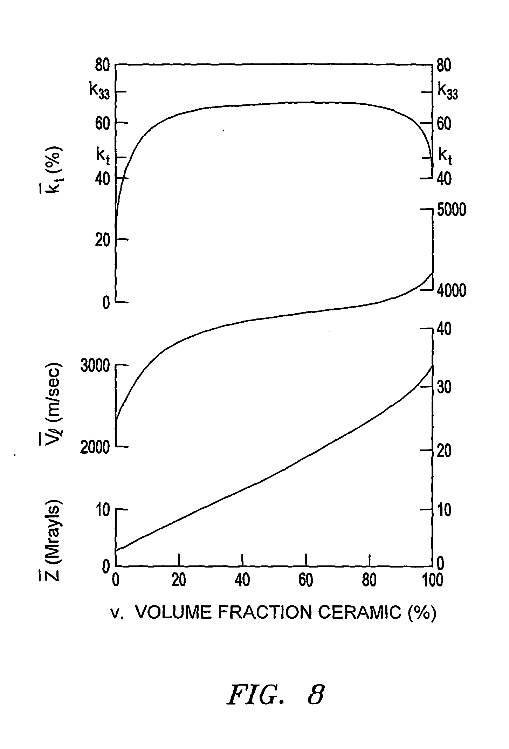

The piezocomposite transducer 106 is preferably composed of a piezoceramic material and a polymer or polymeric material. The use of a piezocomposite material allows the transducer 106 to achieve improved imaging performance. For instance, piezocomposite materials have a lower acoustic impedance and higher electric-mechanical coupling coefficient, kₜ, than piezoceramic materials alone. Since the acoustic impedance is lower, the degree of impedance mismatch between the piezocomposite transducer 106 and the surrounding environment is less than transducers employing piezoceramic materials alone. Also, the higher coupling coefficient, kₜ, allows the piezocomposite transducer 106 to be configured to operate over a wider bandwidth of ultrasound energy and/or to operate with a greater sensitivity to ultrasound energy. The performance characteristics of the piezocomposite transducer 106 are discussed in more detail below with respect to FIGs. 8-9B.

The type of piezoceramic material used to form the piezocomposite material can include PZT type piezoceramics, such as PZT-5A, PZT-7A, PZT-8 and PZT-5H. The polymeric material used to form the piezocomposite material can include most types of epoxy and the like. It should be noted that transducer 106 can use any piezocomposite material formed from any suitable type of piezoceramic and polymeric materials and that transducer 106 is not limited to piezocomposite materials made from any one type of material. In selecting the piezoceramic material, the acoustic impedance, electrical impedance and acoustical properties, among others, should be considered. In selecting the polymeric material, the mechanical and thermal properties, among others, should be considered.

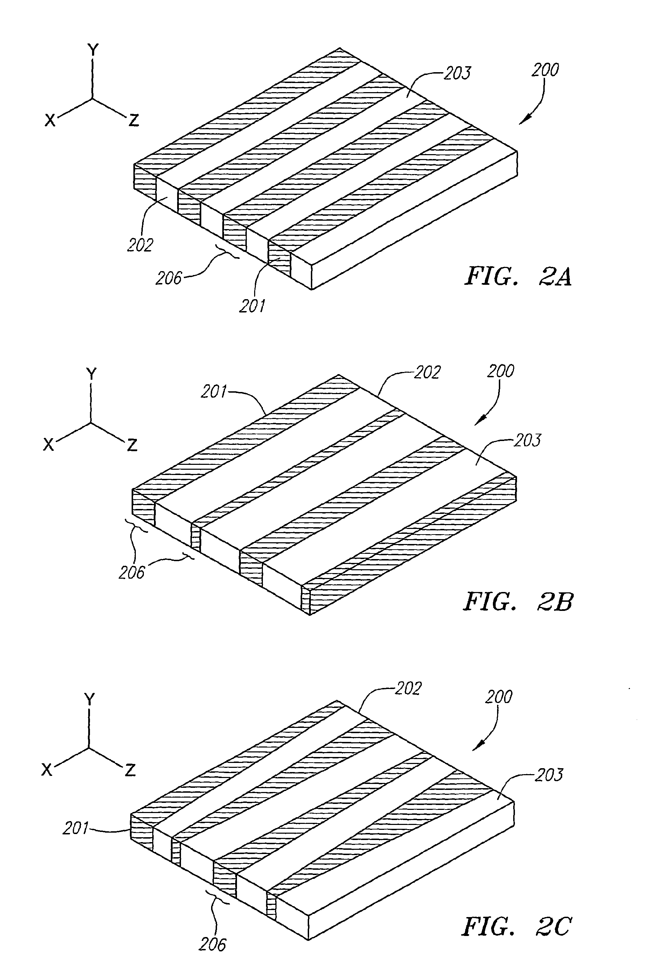

By manipulating the ratio of piezoceramic material to polymeric material, the performance characteristics of a piezocomposite transducer 106 can be adjusted. Due to the nature of the constituent materials, the piezocomposite material is preferably formed from sections of piezoceramic material intermixed with sections of polymeric material, although the materials can be blended together as well. FIGs. 2A-C are perspective views depicting several example embodiments of a piezocomposite material 200. In FIG. 2A, the piezocomposite material 200 includes multiple elongate sections 201 of piezoceramic material arranged with multiple elongate sections 202 of polymeric material. In this embodiment, surface 203 of the piezocomposite material 200 is the active surface from which ultrasound energy is transmitted and/or received. The elongate sections 201 and 202 are arranged such that the active surface 203 preferably intersects each elongate section 201 and 202. Preferably, a portion of each section 201 and 202 is exposed on this surface 203.

In the embodiment depicted in FIG. 2A, each section 201 and 202 has a thickness 206 in the Z direction. In this embodiment, width 206 is preferably uniform, or non-varying, across the entire section 201 and 202 in the Y direction. Also, in this embodiment, the thickness 206 of each section 201 or 202 is the same or very similar, resulting in a volume ratio 0.5. It should be understood that the volume ratio can be adjusted and elongate sections 201 and 202 can be configured in any manner in accordance with the needs of the application. For instance, each elongate section 201 or 202 can have a different thickness 206 as depicted in FIG 2B. Also, each elongate section 201 and 202 can have a varying thickness 206 as depicted in FIG. 2C. In this embodiment, the volume ratio of piezoceramic material to polymeric material is preferably adjusted by adjusting the thickness of each section 201 relative to each section 202.

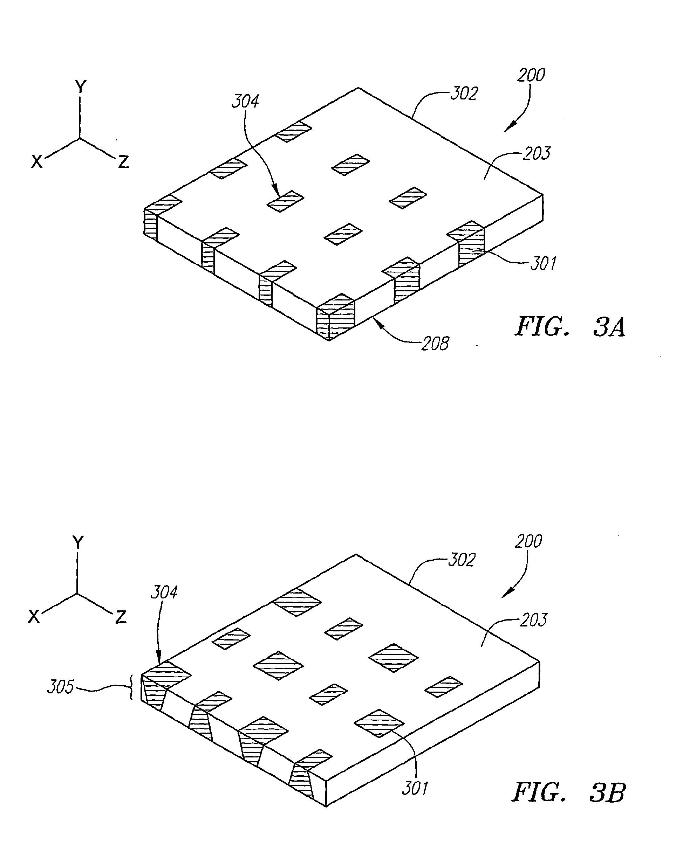

FIGs. 3A-C are perspective views depicting additional example embodiments of the piezocomposite material 200. In FIG. 3A, the piezocomposite material 200 includes multiple sections 301 of piezoceramic material distributed within a base section 302 of polymeric material. In this embodiment, Me sections 301 are preferably configured as columns extending from the active surface 203 to the backside 208. Although here each column 301 has a square cross section 304, it should be noted that the columns can be shaped or configured in any manner in accordance with the needs of the invention. For instance, columns 301 can have a round cross section, a polygonal cross section, a rectangular cross section, any combination thereof and other types of cross sections.

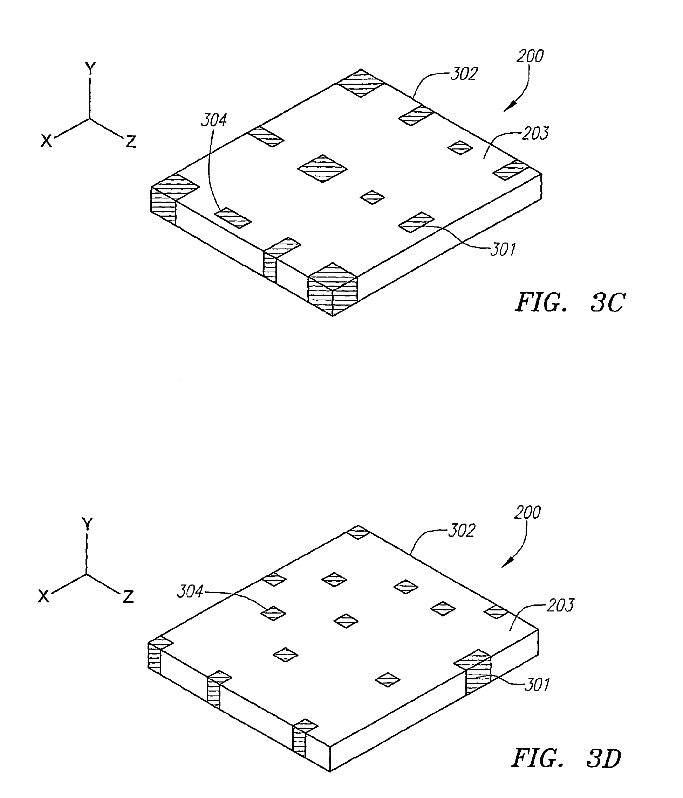

In this embodiment, each section 301 has a similarly sized cross section 304. However, the size or volume of each section 301, and the overall number of sections 301 can be adjusted in any manner in accordance with the needs of the application. For instance, each section 301 can have a varying thickness cross section 304 over it's length 305 as depicted in FIG 3B. Also, each section 301 can have a differently sized cross section 304 as depicted in FIG. 3C. Furthermore, the sections 301 can be distributed within base section 302 in an irregular manner as depicted in FIG. 3D. Any configuration can be used to adjust the performance or volume fraction of the piezocomposite transducer 106. Also, the piezoceramic material and the polymeric material can be arranged in any combination of the embodiments described above, including, but not limited to various combinations of layers, sections or columns and so on.

The embodiments depicted in FIGs. 2A-C can be described as a 2-2 configuration, while the embodiments depicted in FIGs. 3A-D can be described as a 1-3 configuration. This description method describes the structure of the piezocomposite material 200 based on the number of directions in which each section of the piezoceramic material and polymeric material mainly extend. The description method preferably uses an M-N labeling convention, where M is the number of directions in which the piezoceramic material mainly extends and N is the number of directions in which the polymeric material mainly extends.

For instance, in FIG. 2A, both the piezoceramic elongate sections 201 and the polymeric elongate sections 202 extend mainly in the X-Y direction. The elongate sections 201 and 202 also extend in the Z direction, but compared with the extension of elongate sections 201 and 202 in the X and Y directions the degree of extension in the Z direction is much less. Similarly, for the 1-3 configuration depicted in FIGs. 3A-D, the sections 301 extend mainly in the Y direction as compared to the X and Z directions. The section 302 extends to the same degree in each of the X, Y and Z directions. Thus, because the piezoceramic material extends mainly in one direction and the polymeric material extends mainly in three directions, this configuration is referred to as a 1-3 configuration.

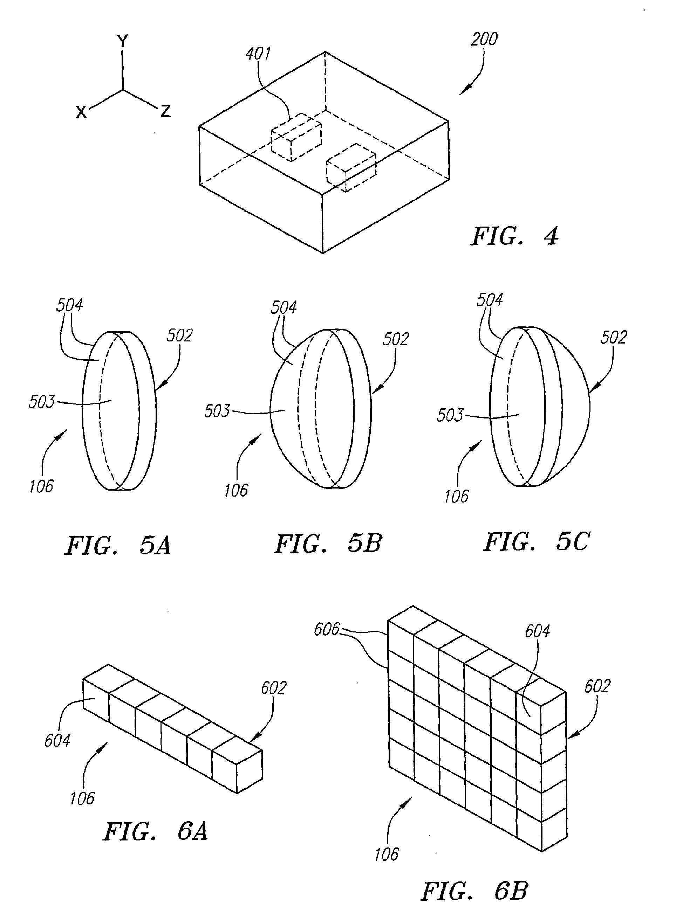

One of skill in the art will readily recognize that the piezocomposite material 200 can also be configured in a 0-3 configuration as depicted in FIG. 4. Here, the sections 401 of piezoceramic material are encapsulated as nodes within the polymeric section 402. To show this, each section 401 is represented by a dotted line. Because the sections 401 do not extend in any direction to a substantial degree, this configuration can be described as a 0-3 configuration. It should be noted that sections 401 can have any shape in accordance with the needs of the application and are not limited to the cube-like shape depicted here.

The piezoelectric transducer 106 can be configured as a single element transducer, an array or any other configuration desired. FIGs. 5A-7B depict multiple example embodiments of transducer 106 in various single element and array configurations. FIG. 5A depicts one example embodiment of the piezocomposite transducer 106 configured to operate in thickness mode as a single element plate 502. In this embodiment, the plate 502 is substantially flattened or planar and configured to transmit or receive ultrasound energy from surface 503. However, because the piezocomposite material is not as fragile and is less resistant to physical manipulation than transducers fabricated from mainly piezocomposite materials, the plate 502 can also be shaped or curved in any number of directions and in any manner in accordance with the application.

For example, two example embodiments of plate 502 having different shapes are depicted in FIGs. 5B-C. FIG. 5B depicts an example embodiment where plate 502 is shaped or curved in a convex manner and FIG. 5C depicts an example embodiment where plate 502 is shaped or curved in a concave manner. The shaping of plate 502 can, for instance, adjust the physical focus of the transducer 106 to allow imaging of a various different ranges of depths or focal points. Plate 502 can also be shaped or pressed into other symmetric or asymmetric shapes as desired.

The outer edge portion 504 of the embodiments of plate 502 depicted in FIGs. 5A-C is rounded. It should be noted that the outer edge portion 504 of plate 502 can have any other desired shape. For instance, the outer edge portion 504 can be substantially oval, asymmetric, symmetric, irregular, polygonal, such as square, hexagonal, octagonal, or any combination thereof or any other shape. The use of the term "substantially" in the preceding sentence means that outer edge portion 504 does not need to be absolutely oval, asymmetric, symmetric etc. For instance, a substantially square outer edge portion 504 might have rounded corners between each of the four sides and so forth.

As mentioned above, transducer 106 can also be configured as an array. FIG. 6A depicts an example embodiment of transducer 106 configured as a linear, or one dimensional (1D), array 602. In this embodiment, the array 602 includes multiple transducer elements 604 arranged in a row. Preferably, each element 604 in the array 602 is composed of a piezocomposite material, although transducer elements composed of different materials can be used in combination with one or more piezocomposite elements 604. Preferably, each element 604 is electrically coupled to an image processing system for processing image data collected by each element 604. It should be noted that array 602 can also be configured as a single element 604 with multiple electrodes coupled along the element 604 in order to operate as an array.

FIG. 6B depicts another example embodiment of transducer 106 configured as a two dimensional (2D) array 602. A 2D array 602 preferably includes M rows 606 of elements 604, each row having M elements 604 within it, where M is any number in accordance with the needs of the application. In FIG. 6B array 602 is depicted having 5 rows 606 with 5 elements per row 606. Array 602 can have any number of rows 606 and elements per row 606 as desired. Array 602 can also be configured with varying numbers of elements 604 in each row 606 if desired.

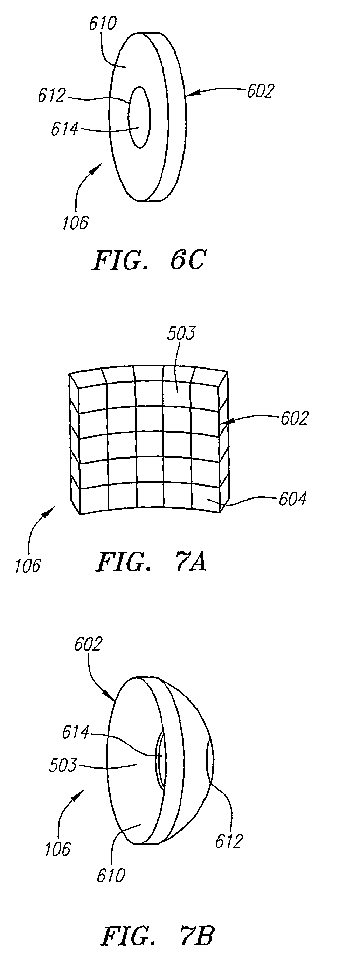

FIG. 6C depicts another example embodiment where array 602 has an annular configuration of elements 604. In this embodiment, array 602 has an outer element 610 with an aperture 612. A second element 614 is configured to fit within the aperture 612. In this embodiment, the outer element 610 is circular and concentric, although it should be noted that the array can be configured in any manner in accordance with the needs of the application. For instance, array 602 can have more than two elements and can be arranged eccentrically or have a polygonal shape, oval shape or other shapes.

Similar to the embodiments discussed above with respect to FIGs. 5B-C, array 602 can be shaped in accordance with the needs of the application. FIG. 7A depicts a 2D array 602 having a curved shape and FIG. 7B depicts an annular array 602 having a curved concave shape. Although not shown, each of the configurations of transducer 106 depicted in FIGs. 5A-7B can include one or more matching layers if desired. The use of matching layers is well known to one of skill in the art. It should be noted that the example embodiments depicted in FIGs. 5A-7B are example embodiments intended only to aid in the illustration of the various configurations of transducer 106. Since a large number of configurations are possible, it is not intended that every possible shape of configuration be described or depicted and, therefore, piezocomposite transducer 106 should not be limited to any one shape or configuration described or depicted herein.

FIG. 8 depicts a graph of the performance characteristics of one example embodiment of piezocomposite material in a 1-3 configuration, specifically, the coupling coefficient for thickness mode operation, kₜ, the longitudinal sound velocity, V1, and the acoustic impedance Z versus the ceramic to polymer volume fraction. Here, it can be seen that the coupling coefficient, kₜ, for the piezocomposite material approaches 0.7 in the volume fraction range of 30-60%, while at the same time the acoustic impedance is in the range of 12-17 MRayl. This is a significant improvement over piezoceramics alone, which have a kₜ of approximately 0.5 and an acoustic impedance of greater than 30 MRayl.

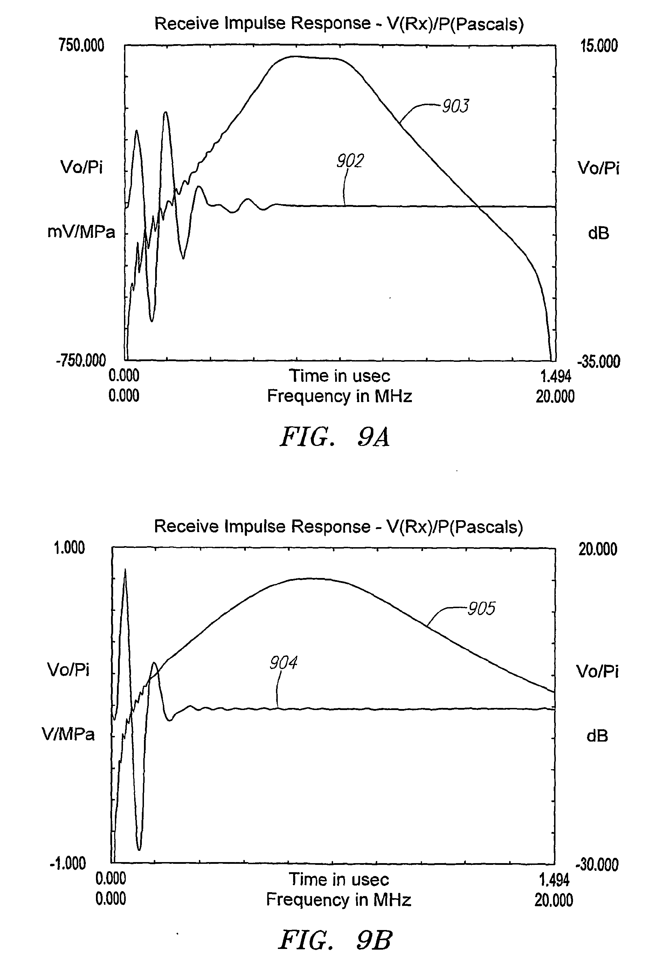

FIG. 9A depicts simulated graphs of the time domain response 902 and the frequency domain response 903 of an example piezoceramic transducer, while FIG. 9B depicts simulated graphs of the time domain response 904 and the frequency domain response 905 of an example embodiment of the piezocomposite transducer 106. In each graph, the simulated transducer is a single element transducer having a diameter of 1.93 millimeters (mm) and a center frequency of 9 Megahertz (Mhz). Transducer 106 simulated in FIG. 9B has a 35% volume fraction.

In FIG. 9A, the piezoceramic transducer has a pulse length of approximately 3 cycles in time domain response 902, as indicated by the number of oscillations in the response 902, and the peak-to-peak voltage of the piezoceramic transducer is approximately 1.05 Volts. FIG. 9B demonstrates the improved performance of the piezocomposite transducer 106. Here, transducer 106 has a pulse length of approximately 1.5 cycles in time domain response 904, as indicated by the number of oscillations in the response 904, and the peak-to-peak voltage of transducer 106 is approximately 1.9 Volts. Furthermore, the piezocomposite transducer 106 of FIG. 9B has a much larger bandwidth than the piezoceramic transducer of FIG. 9A, as indicated by a comparison of frequency responses 905 and 903.

It should be noted that piezocomposite transducer 106 can be configured to operate at frequencies higher than those depicted in FIGs. 9A-B. For instance, in some ICE applications, piezocomposite transducer 106 can be configured to operate at a center frequency of approximately 10 Mhz, while in some IVUS applications, piezocomposite transducer 106 can be configured to operate at a center frequency in the range of 20-50 Mhz. Piezocomposite transducer 106 can be configured to operate at any frequency in accordance with the needs of the application and should not be limited to any one frequency range described herein.

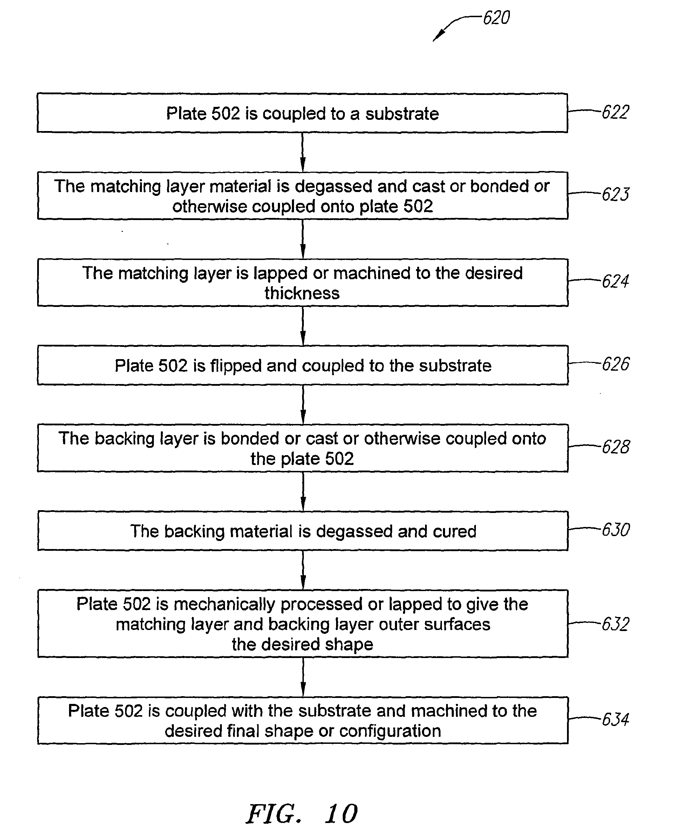

Also provided herein are methods 620 of manufacturing a single element piezocomposite transducer 106. These methods are not part of the invention. Methods 620 can include three main steps: bonding or casting a matching layer; bonding or cast a backing layer; and machining the transducer 106 to the desired shape or dimensions. FIG. 10 depicts an example manufacturing method 620 for a single element piezocomposite transducer 106. Before beginning method 620, the piezocomposite material is preferably provided in the form of a plate 502 having the desired thickness, which can determine the resonant frequency of the transducer 106. The plate 502 also preferably has an electrode disposed on the front and back sides. Because piezocomposite materials have lower acoustic impedances than piezoceramic materials, the use of matching layers are not as important. However, it still can be desirable to include the matching layers. For instance, a matching layer having an acoustic impedance of approximately 4-5 MRayl will enhance the acoustic coupling between the piezocomposite material and the surrounding environment.

Referring to method 620, to form the optional matching layer, the plate 502 is first coupled to a substrate, such as a glass plate at 622. Then, at 623, the matching layer material, such as a solgel-like material, can be degassed and cast or bonded or otherwise coupled onto plate 502. At 624, after the matching layer material has cured, the matching layer can be lapped or machined to the desired thickness. Typically, the matching layer has a thickness of one quarter wavelength of the ultrasound wave at the working, or operational, frequency.

Next, to form the backing layer, the plate is flipped at 626 and coupled again to the substrate. If the plate 502 is to be focused or shaped, the substrate preferably has a reciprocal shape that can be used to press the desired shape onto the plate 502. At 628, the backing layer is bonded or cast or otherwise coupled onto the plate 502. The backing layer is located on the backside of the plate 502 and provides mechanical support for the transducer 106 and attenuates all acoustic energy that propagates backward. The thickness of the backing layer is preferably adequate to meet the desired amount of acoustic absorption. One example thickness for a backing layer is 5 mm, although any thickness can be used. Excess backing layer can serve as a sacrificial substrate for later mechanical processing. At 630, the backing material is degassed and cured. If a large amount of backing material is bonded or cast, a mold can be used to hold the plate 502 during degas and curing.

At 632, the plate 502 is mechanically processed or lapped to give the matching layer and backing layer outer surfaces the desired shape. Preferably, the matching layer and backing layer outer surfaces are made as parallel as possible. At 634, the plate 502 is coupled with the substrate and machined to the desired final shape or configuration. For instance, the outer edge portion 504 can be machined, or diced, to a polygonal configuration. It should be noted that method 620 is one example method of manufacturing, and that piezocomposite transducer 106 is not limited to manufacture only with method 620. Other methods, including, but not limited to the use of dicing, filling, molding random fibers, and composite films can be used. Although methods 620 applies to single element transducers 106, piezocomposite transducer 106 is not limited to single element transducers and can include transducer arrays having one or more transducer elements and other transducer configurations.

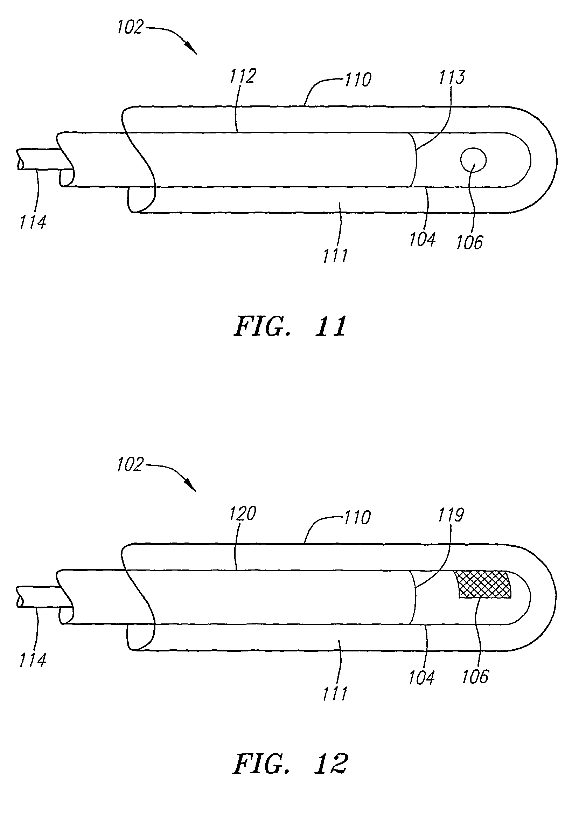

Also provided herein is an example method 640 of imaging with a piezocomposite transducer 106. FIG. 11 depicts an example embodiment of medical device 102 suitable for use with method 640. Medical device 102 can be any device insertable into a living being including, but not limited to a catheter and an endoscope. In this embodiment, medical device 102 includes a flexible elongate tubular member 110 with an inner lumen 111. Imaging device 104 is coupled with the distal end 113 of a flexible elongate driveshaft 112. Inner lumen 111 is preferably configured to slidably receive driveshaft 112 and allow driveshaft 112 and imaging device 104 to rotate therein. The piezocomposite transducer 106 can be configured as a single element transducer communicatively coupled via one or more signal lines 114 to an image processing system (not shown). The interior of the living being is preferably imaged by rotating driveshaft 112 while imaging with piezocomposite transducer 106.

FIG. 12 depicts another example embodiment of medical device 102 suitable for use with method 640. In this embodiment, medical device 102 includes flexible elongate tubular member 110 with an inner lumen 111. Imaging device 104 is coupled with the distal end 119 of a flexible elongate member 120. Inner lumen 111 is preferably configured to slidably receive member 120 and allow member 120 to move therein. The piezocomposite transducer 106 can be configured as an array communicatively coupled via one or more signal lines 114 to an image processing system (not shown). The interior of the living being is preferably imaged with array 106, which can be positioned and repositioned by moving member 120 within inner lumen 111. It should be noted that the embodiments depicted in FIGs. 11 and 12 are example embodiments and are not intended to limit medical device 102 or piezocomposite transducer 106. Other embodiments of medical device 102 can also be used with piezocomposite transducer 106.

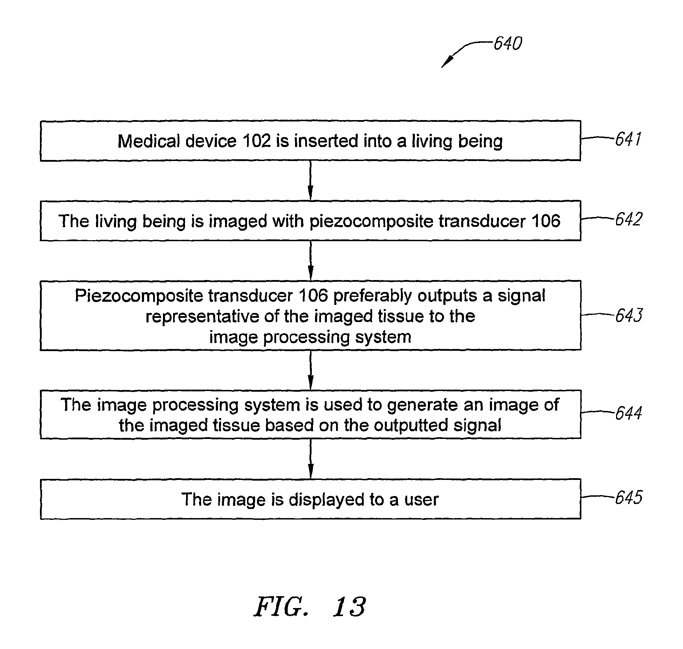

FIG. 13 depicts an example of imaging method 640, which can be performed with embodiments of medical device 102 similar to those discussed with respect to FIGs. 11-12. First, at 641, medical device 102 is inserted into a living being. At 642, the living being is imaged with piezocomposite transducer 106. If piezocomposite transducer 106 is a single element transducer 106 similar to that discussed with respect to FIG. 11, step 642 preferably includes rotating imaging device 104 within inner lumen 111 while imaging. If piezocomposite transducer 106 is an array similar to that discussed with respect to FIG. 12, step 642 can be performed without rotating imaging device 104 and an image can be generated in a manner in accordance with the configuration of array 602, such as 1D, 2D, linear or phased configurations and the like.

Next at 643, piezocomposite transducer 106 preferably outputs a signal representative of the imaged tissue to the image processing system. At 644, the image processing system can be used to generate an image of the imaged tissue based on the outputted signal. At 645, the image can be displayed to a user.

In the foregoing specification, the invention has been described with reference to specific embodiments thereof. It will, however, be evident that various modifications and changes may be made thereto without departing from the broader scope of the invention. For example, each feature of one embodiment can be mixed and matched with other features shown in other embodiments, and the sequence of steps shown in a flowchart may be changed. Features and processes known to those of ordinary skill may similarly be incorporated as desired. Additionally and obviously, features may be added or subtracted as desired. Accordingly, the invention is not to be restricted except in light of the attached claims and their equivalents.

## Claims

1. An ultrasound imaging apparatus, comprising:
an imaging device insertable into a living being and configured to image the interior of the living being, the imaging device comprising a piezocomposite transducer (106), **characterized in that** the piezocomposite transducer is configured to transmit ultrasound energy from a flat surface and comprises a plurality of sections (201,301) of piezoceramic material either a) intermixed with a plurality of sections of polymeric material or b) distributed within a base section of polymeric material, wherein each section of piezoceramic material has a varying thickness over its length.

2. The apparatus of claim 1, wherein the transducer (106) has a center frequency in a range from 20 to 50 MHz.

3. The apparatus of claim1, wherein the piezoceramic material and polymeric material are arranged in a plurality of elongate sections.

4. The apparatus of claim 3, wherein the piezoceramic material and polymeric material include a 2-2 configuraton.

5. The apparatus of claim 1, wherein the piezoceramic material is arranged as a plurality of sections located within the base section of the polymeric material.

6. The apparatus of claim 5, wherein the sections (201, 301) of piezoceramic material are configured as columns.

7. The apparatus of claim 5, wherein the piezoceramic material and polemeric material include a 1-3 configuration.

8. The apparatus of claim 1, wherein the piezoceramic material is arranged as a plurality of nodes, at least one node being encapsulated within the polymeric material.

9. The apparatus of claim 8, wherein the piezoceramic material and the polymeric matrial include a 0-3 configuration.

10. The apparatus of claim 1, wherein the piezoceramic material and polymeric material are arranged in a combincation of 2-2 and 1-3 configurations.

11. The apparatus of claim 1, wherein the transducer has one or more matching layers.

12. The apparatus of claim 1, wherein the transducer is a single element transducer (106).

13. The apparatus of claim 12, wherein the single element transducer (106) is configured as a plate.

14. The apparatus of claim 13, wherein the outer edge of the plate is curved or substantially polygonal,

15. The apparatus of claim 14, wherein the outer edge of the plate is substantially square, substantially hexagonal, or substantially octagonal.

16. The apparatus or claim 13, wherein the outer edge of the plate is partially curved and partially straight.

17. The apparatus of claim 1, wherein the transducer (106) is configured as an array.

18. The apparatus of claim 17, wherein the transducer (106) is coupled with a plurality of electrodes.

19. The apparatus of claim 17, wherein the transducer comprises a plurality of transducer elements.

20. The apparatus of claim 19, wherein the plurality of transducer elements are coupled together.

21. The apparatus of claim 19, wherein the plurality of transducer elements are arranged in a row.

22. The apparatus of claim 21, wherein the array is a one dimensional array.

23. The apparatus of claim 19, wherein the plurality of transducer elements are arranged in a plurality of rows, each row comprising a plurality of transducer elements.

24. The apparatus of claim 23, wherein the plurality of transducer elements are arranged in M rows, wherein M transducer elements are located in each row.

25. The apparatus of claim 23, wherein the array is a two dimensional array.

26. The apparatus of claim 19, wherein the array comprises a first transducer element having an aperture (612) and a second transducer element located within the aperture.

27. The apparatus of claim 19, wherein the array (602) is an annular array.

28. The apparatus of claim 27, wherein the transducer elements are arranged concentrically.

29. The apparatus of claim 17, wherein a first surface of the array is configured to transmit ultrasound energy.

30. The apparatus of claim 17, wherein the array is a linear array.

31. The apparatus of claim 17, wherein the array is a phased array.

## Patentansprüche

1. Ultraschall-Abbildungsgerät, welches aufweist:
eine Abbildungsvorrichtung, die in ein lebendes Wesen einführbar und ausgebildet ist, das Innere des lebenden Wesens abzubilden, welche Abbildungsvorrichtung einen Piezoverbundwandler (106) aufweist, **dadurch gekennzeichnet, dass** der Piezoverbundwandler ausgebildet ist zum Aussenden von Ultraschallenergie von einer flachen Oberfläche und mehrere Abschnitte (201, 301) aus piezokeramischem Material aufweist, die entweder a) mit mehreren Abschnitten aus Polymermaterial vermischt sind oder b) innerhalb eines Basisabschnitts aus Polymermaterial verteilt sind, wobei jeder Abschnitt aus piezokeramischem Material eine sich über seine Länge verändernde Dicke hat.

2. Gerät nach Anspruch 1, bei dem der Wandler (106) eine Mittenfrequenz in einem Bereich von 20 bis 50 MHz hat.

3. Gerät nach Anspruch 1, bei dem das piezokeramische Material und das Polymermaterial in mehreren länglichen Abschnitten angeordnet sind.

4. Gerät nach Anspruch 3, bei dem das piezokeramische Material und das Polymermaterial eine 2-2-Konfiguration enthalten.

5. Gerät nach Anspruch 1, bei dem das piezokeramische Material als mehrere Abschnitte, die sich innerhalb des Basisabschnitts des Polymermaterials befinden, angeordnet ist.

6. Gerät nach Anspruch 5, bei dem die Abschnitte (201, 301) aus piezokeramischem Material als Säulen ausgebildet sind.

7. Gerät nach Anspruch 5, bei dem das piezokeramische Material und das Polymermaterial eine 1-3-Konfiguration enthalten.

8. Gerät nach Anspruch 1, bei dem das piezokeramische Material als mehrere Knoten angeordnet ist, wobei zumindest ein Knoten innerhalb des Polymermaterials eingekapselt ist.

9. Gerät nach Anspruch 8, bei dem das piezokeramische Material und das Polymermaterial eine 0-3-Konfiguration enthalten.

10. Gerät nach Anspruch 1, bei dem das piezokeramische Material und das Polymermaterial in einer Kombination aus 2-2- und 1-3-Konfiguration angeordnet sind.

11. Gerät nach Anspruch 1, bei dem der Wandler eine oder mehrere Anpassungsschichten hat.

12. Gerät nach Anspruch 1, bei dem der Wandler ein Einzelelementwandler (106) ist.

13. Gerät nach Anspruch 12, bei dem der Einzelelementwandler (106) als eine Platte ausgebildet ist.

14. Gerät nach Anspruch 13, bei dem die äußere Kante der Platte gekrümmt oder im Wesentlichen polygonal ist.

15. Gerät nach Anspruch 14, bei dem die äußere Kante der Platte im Wesentlichen quadratisch, im Wesentlichen sechseckig oder im Wesentlichen achteckig ist.

16. Gerät nach Anspruch 13, bei dem die äußere Kante der Platte teilweise gekrümmt und teilweise geradlinig ist.

17. Gerät nach Anspruch 1, bei dem der Wandler (106) als eine Gruppierung ausgebildet ist.

18. Gerät nach Anspruch 17, bei dem der Wandler (106) mit mehreren Elektroden gekoppelt ist.

19. Gerät nach Anspruch 17, bei dem der Wandler mehrere Wandlerelemente aufweist.

20. Gerät nach Anspruch 19, bei dem die mehreren Wandlerelemente miteinander gekoppelt sind.

21. Gerät nach Anspruch 19, bei dem die mehreren Wandlerelemente in einer Reihe angeordnet sind.

22. Gerät nach Anspruch 21, bei dem die Gruppierung eine eindimensionale Gruppierung ist.

23. Gerät nach Anspruch 19, bei dem die mehreren Wandlerelemente in mehreren Reihen angeordnet sind, wobei jede Reihe mehrere Wandlerelemente aufweist.

24. Gerät nach Anspruch 23, bei dem die mehreren Wandlerelemente in M Reihen angeordnet sind, wobei M Wandlerelemente in jeder Reihe angeordnet sind.

25. Gerät nach Anspruch 23, bei dem die Gruppierung eine zweidimensionale Gruppierung ist.

26. Gerät nach Anspruch 19, bei dem die Gruppierung ein erstes Wandlerelement mit einer Öffnung (612) und ein zweites Wandlerelement, das sich innerhalb der Öffnung befindet, aufweist.

27. Gerät nach Anspruch 19, bei dem die Gruppierung (602) eine ringförmige Gruppierung ist.

28. Gerät nach Anspruch 27, bei dem die Wandlerelemente konzentrisch angeordnet sind.

29. Gerät nach Anspruch 17, bei dem eine erste Oberfläche der Gruppierung zum Senden von Ultraschallenergie ausgebildet ist.

30. Gerät nach Anspruch 17, bei dem die Gruppierung eine lineare Gruppierung ist.

31. Gerät nach Anspruch 17, bei dem die Gruppierung eines phasengesteuerte Gruppierung ist.

## Revendications

1. Appareil d'imagerie ultrasons, comprenant :
un dispositif d'imagerie pouvant être inséré dans un être vivant et configuré pour imager l'intérieur de l'être vivant, le dispositif d'imagerie comprenant un transducteur piézocomposite (106), **caractérisé en ce que** le transducteur piézocomposite est configuré pour émettre de l'énergie ultrasonore depuis une surface plane et comprend une pluralité de sections (201, 301) en matériau piézocéramique soit a) intermélangées avec une pluralité de sections en matériau polymérique, soit b) distribuées à l'intérieur d'une section de base en matériau polymérique, dans lequel chaque section en matériau piézocéramique présente une épaisseur variable sur sa longueur.

2. Appareil selon la revendication 1, dans lequel le transducteur piézocomposite (106) présente une fréquence centrale dans une plage de 20 à 50 MHz.

3. Appareil selon la revendication 1, dans lequel le matériau piézocéramique et le matériau polymérique sont agencés selon une pluralité de sections allongées.

4. Appareil selon la revendication 3, dans lequel le matériau piézocéramique et le matériau polymérique incluent une configuration 2-2.

5. Appareil selon la revendication 1, dans lequel le matériau piézocéramique est agencé en une pluralité de sections situées à l'intérieur de la section de base du matériau polymérique.

6. Appareil selon la revendication 5, dans lequel les sections (201, 301) en matériau piézocéramique sont configurées en colonnes.

7. Appareil selon la revendication 5, dans lequel le matériau piézocéramique et le matériau polymérique incluent une configuration 1-3.

8. Appareil selon la revendication 1, dans lequel le matériau piézocéramique est agencé en une pluralité de noeuds, au moins un noeud étant encapsulé dans le matériau polymérique.

9. Appareil selon la revendication 8, dans lequel le matériau piézocéramique et le matériau polymérique incluent une configuration 0-3.

10. Appareil selon la revendication 1, dans lequel le matériau piézocéramique et le matériau polymérique sont agencés selon une combinaison de configurations 2-2 et 1-3.

11. Appareil selon la revendication 1, dans lequel le transducteur comporte une ou plusieurs couches d'adaptation.

12. Appareil selon la revendication 1, dans lequel le transducteur est un transducteur à un seul élément.

13. Appareil selon la revendication 12, dans lequel le transducteur à un seul élément (106) est configuré en tant que plaque.

14. Appareil selon la revendication 13, dans lequel le bord externe de la plaque est incurvé ou sensiblement polygonal.

15. Appareil selon la revendication 14, dans lequel le bord externe de la plaque est sensiblement carré, sensiblement hexagonal ou sensiblement octogonal.

16. Appareil selon la revendication 13, dans lequel le bord externe de la plaque est partiellement incurvé et partiellement rectiligne.

17. Appareil selon la revendication 1, dans lequel le transducteur (106) est configuré en tant que réseau.

18. Appareil selon la revendication 17, dans lequel le transducteur (106) est couplé avec une pluralité d'électrodes.

19. Appareil selon la revendication 17, dans lequel le transducteur comprend une pluralité d'éléments de transducteur.

20. Appareil selon la revendication 19, dans lequel la pluralité d'éléments de transducteur sont couplés ensemble.

21. Appareil selon la revendication 19, dans lequel la pluralité d'éléments de transducteur sont agencés selon une rangée.

22. Appareil selon la revendication 21, dans lequel le réseau est un réseau monodimensionnel.

23. Appareil selon la revendication 19, dans lequel la pluralité d'éléments de transducteur sont agencés selon une pluralité de rangées, chaque rangée comprenant une pluralité d'éléments de transducteur.

24. Appareil selon la revendication 23, dans lequel la pluralité d'éléments de transducteur sont agencés selon M rangées, dans lequel M éléments de transducteur sont situés dans chaque rangée.

25. Appareil selon la revendication 23, dans lequel le réseau est un réseau bidimensionnel.

26. Appareil selon la revendication 19, dans lequel le réseau comprend un premier élément de transducteur comportant une ouverture (612) et un second élément de transducteur situé à l'intérieur de l'ouverture.

27. Appareil selon la revendication 19, dans lequel le réseau (602) est un réseau annulaire.

28. Appareil selon la revendication 27, dans lequel les éléments de transducteur sont agencés de façon concentrique.

29. Appareil selon la revendication 17, dans lequel une première surface du réseau est configurée pour émettre de l'énergie ultrasonore.

30. Appareil selon la revendication 17, dans lequel le réseau est un réseau linéaire.

31. Appareil selon la revendication 17, dans lequel le réseau est un réseau phasé.
